# EUROPEAN PATENT APPLICATION

(11) **EP 1 702 634 A1**
(43) Date of publication of application: **20.09.2006**
(21) Application number: 04807792.9
(22) Date of filing: 24.12.2004
(51) Int. Cl.: A61M 3/00, A61M 31/00

(54) **PORTABLE EJECTION AND INJECTION DEVICE**

(30) Priority: 26.12.2003 JP 2003435256; 26.12.2003 JP 2003435270; 21.01.2004 JP 2004013164; 19.02.2004 JP 2004043569; 19.02.2004 JP 2004043573; 19.02.2004 JP 2004043578
(71) Applicant: Wet Trust Japan Co., Ltd., Osaka-shi, Osaka 542-0081 (JP)
(72) Inventor: TAKESHITA, Masato, c/o Wet Trust Japan Co., Ltd., Osaka-shi, Osaka 542-0081 (JP); BANG, Ji-Hwan, c/o JTB Co., Ltd., Seongnam-City, Kyeonggi-Do (KR)
(74) Representative: Calderbank, Thomas Roger
(86) International application number: PCT/JP2004/019436
(87) International publication number: WO 2005/070479

(57) **Abstract**

A portable ejection and injection device has a cylinder, a piston member and a pushing rod. The cylinder includes an ejection nozzle at its tip portion and a filling chamber to contain a liquid medicine or a lubricant. The piston member is supported within the cylinder and seals the liquid medicine or the lubricant filled in the filling chamber. The pushing rod is to be inserted into the cylinder, and formed integrally with or separately from the piston member. The cylinder and the piston member are pushed inward to eject the liquid medicine or the lubricant out and inject it in directly or by air pressure produced in the cylinder. With the portable ejection and injection device, a liquid medicine or a lubricant may be ejected to the genital area of a woman or the anus of a person to clean such a area or directly injected in it as required.

## Description

### Technical Field of the Invention

The present invention relates to a portable ejection and injection device of a liquid medicine or a lubricant for personal use, in particular, a portable ejection and injection device having a chamber that contains a certain liquid medicine or a lubricant in advance through which the medicine or lubricant is ejected to and injected into a specific body portion such as a female genital organ or a person's anus. The manner of ejection of medicine and lubricant includes from slowly flowing out to vividly running (jetting) out from the device. The liquid medicine includes contraceptives, anesthetics, enemas, antipyretics and the like, and the lubricant includes lubricant lotions, lubricant jells, lubricant jellies and the like. Selection thereof could be made according to the purposes.

### Description of the Prior Art

In the present days people lead complicated and continuously busy life. In order to live through such present days, various devices have been developed to solve problems exist in present busy daily life. Notwithstanding, handy and hygienic devices for inserting medical substances into a specific body portion for the purpose of women's contraceptive or medical treatment have not been developed sufficiently. Although maintaining sanitariness around the genital organs (the vicinity of vagina) and keeping cleanness around a person's anus are very important for prevention from diseases, injection of contraceptives and injection of specific medical substances such as enema and the like have been directly made by human's hands. And such injections by hand are rather troublesome and not hygienic.

Since a female genital organs (a vagina) and a person's anus (inside the anus) are very sensitive and delicate portions, lubricants, contraceptives, medicines for treatment of venereal diseases, enemas and the like must be carefully injected into these portions.

However, handy and safe devices for effectively cleaning a female genital organ and a person's anus and its vicinity and injecting a specific medical liquid or a lubricant thereinto have not been developed yet. The reasons are assumed that such devices need to have portability in all occasions independent of time and place and use of such devices are very personal.

One example of this type of medicine injection apparatus is described in Japanese Patent Application 2001-187151A. This injection apparatus has a plastic cylinder having an outlet of lubricant at the tip and a pushing rod therein, in which the pushing rod is pushed toward the outlet of lubricant to let the lubricant filled in the tip portion of the cylinder out through the outlet. With this device, lubricant is relatively easily injected into the female genital organ.

The foregoing apparatus, however, have no measures against leakage of lubricant, and therefore lubricant is likely to flow or ooze out from the outlet while being carried around and the apparatus does not have high and suitable portability. The apparatus can also be reused, which is insanitary and liable to cause infection of diseases. Furthermore, the apparatus being held upright or obliquely upward, the pushing rod must be pushed upwardly from below and the device is not readily used. Prior Art Reference 1: Japanese Patent Application / Publication 2001-187151A

### Summary of the Invention

### Problems to be solved by the Invention

It is hence an object of the present invention to provide a portable ejection and injection device with which a female genital organ and an inside of a person's anus can be cleaned by ejection of a liquid medicine or a lubricant thereto and, if necessary, it can be directly injected thereinto.

It is another object of the present invention to provide a portable ejection and injection device in which the liquid medicine or the lubricant is unlikely to leak from an outlet.

It is another object of the present invention to provide a portable ejection and injection device which is designed against infection of diseases and for hygienic purposes.

It is another object of the present invention to provide a portable ejection and injection device in which inadvertent flow-out is prevented while the device is carrying around.

It is a further object of the present invention to provide a portable ejection and injection device with which a liquid medicine and a lubricant can be readily ejected and injected.

### Means to solve the Problem

A portable ejection and injection device according to the present invention, which ejects a liquid medicine or a lubricant to a female genital organ or a person's anus and its vicinity and injects it thereinto, includes a cylinder with an ejection nozzle provided at the tip portion and a filling chamber for a liquid medicine or a lubricant within the cylinder, a piston member supported within the cylinder and sealing the liquid medicine or the lubricant within the filling chamber and a pushing rod inserted within the cylinder separately from the piston member. Upon receipt of a push-in force, the pushing rod pushes the piston member toward the ejection nozzle. A liquid medicine or a lubricant is ejected out through the ejection nozzle.

The cylinder may be formed with an insertion part and longitudinal part. The insertion part is formed as a chap member having the ejection nozzle, the filling chamber and a easily breakable sealing membrane member to seal a rear end of the filing chamber. The cap member may be removably engaged with a thread with the longitudinal part and the longitudinal part supports the piston member therein.

In the portable ejection and injection device according to the present invention, a closing member may be provided to cover the ejection nozzle, and the piston member and the pushing rod are separate from each other or integral with each other. While the closing member is removed, inward shift of the pushing rod to which a push-in force is applied allows a liquid medicine or a lubricant to spout out through the ejection nozzle.

Furthermore, in the portable ejection and injection device according to the present invention, the pushing rod may be integrally formed with the piston member. The pushing rods is inserted in the cylinder in a manner that at least a part is watertight against the inner wall of the cylinder, and a push-in part projects outwardly to be exposed outside. They cylinder is bent or curved, or capable of being bent or curved, between its length. When a push-in force is applied to the pushing rod, a liquid medicine and a lubricant are is ejected out through the ejection nozzle directly by the pushing rod or due to pressured air within the cylinder.

### Effects of the invention

The present invention being constituted as stated above, with a jet of medicines or lubricants to a female genital organ and a person's anus, these body portions can be cleaned, if necessary, a liquid medicine or a lubricant can be directly injected within these portions (a vagina and an inside of the anus), and such a liquid medicine or a lubricant is unlikely to leak out from the ejection nozzle. While the device being carried around, the liquid medicine or the lubricant flow out is eliminated. Furthermore, not only easy ejection and injection of the liquid medicine or the lubricant but also prevention of disease infection and sanitary use become possible.

### Brief Description of Drawings

Fig. 1 is an exploded perspective view of a portable ejection and injection device according to Embodiment 1 of the present invention.
Fig. 2 is a sectional view of the portable ejection and injection device of Fig. 1; an upper section shows a front side of the portable ejection and injection device and a lower section shows a rear side thereof in which the pushing rods is pushed and an enlarged portion thereof is stuck in a funnel portion of a cylinder..
Fig. 3 is a sectional view showing how the device works.
Fig. 4 is a sectional view of another portable ejection and injection device according to Embodiment 2 of the present invention.
Fig. 5 is an enlarged view of a portion B of Fig. 4.
Fig. 6 is s a sectional view of another portable ejection and injection device according to Embodiment 3 of the present invention.
Fig. 7 is a sectional view of a front tip portion of a portable ejection and injection device according to Embodiment 4 of the present invention.
Fig. 8 is a sectional view of a front tip portion of a portable ejection and injection device similar to Embodiment 4.
Fig. 9 is a sectional view of a front tip portion of another portable ejection and injection device similar to Embodiment 4.

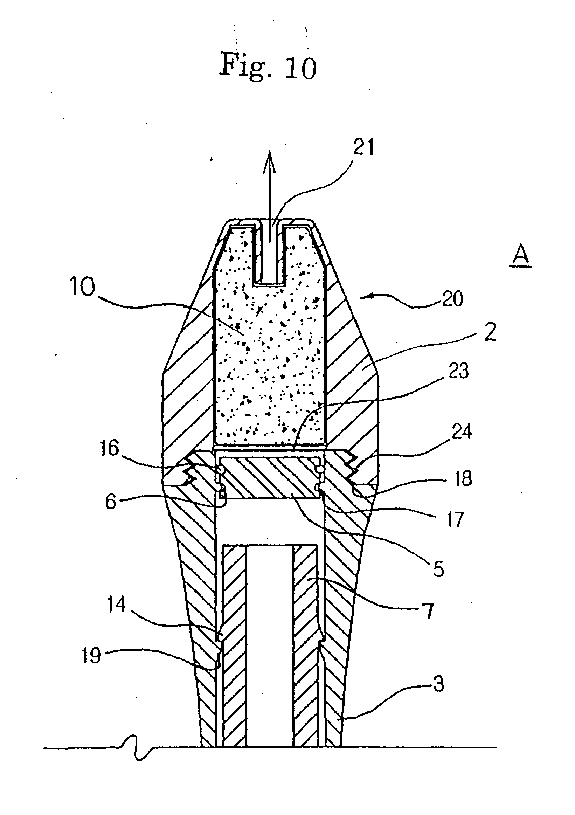

Fig. 10 is a sectional view of a front tip portion of another portable ejection and injection device similar to Embodiment 4.
Fig. 11 is a sectional view of a portable ejection and injection device according to Embodiment 5 of the present invention.
Fig. 12 is an enlarged view of a portion B' of Fig. 11.
Fig. 13 is a sectional view of a portable ejection and injection device according to Embodiment 6 of the present invention, in which a pushing rod is ready to be shifted toward a front tip portion.
Fig. 14 is a sectional view in which the pushing rod of Fig. 13 has been pushed in and a piston member is shifted to or near an ejection nozzle.
Fig. 15 is a sectional view in which the pushing rod is drawn back from the state of Fig. 14.
Fig. 16 is an exploded view of a portable ejection and injection device according to Embodiment 7 of the present invention in which a cylinder and a cap (a means to cover) is illustrated in a sectional view.
Fig. 17 is an exploded view of a portable ejection and injection device according to Embodiment 8 of the present invention in which a cylinder is illustrated in a sectional view.
Fig. 18 is a partial sectional view of a portable ejection and injection device according to Embodiment 9 of the present invention.
Fig. 19 is an exploded view of a portable ejection and injection device according to Embodiment 10 of the present invention in which a cylinder is illustrated in a sectional view.
Fig. 20 is an exploded view of a portable ejection and injection device according to Embodiment 11 of the present invention.
Fig. 21 is an exploded view of a portable ejection and injection device according to Embodiment 12 of the present invention in which a cylinder is illustrated in a sectional view.
Fig. 22 is a sectional view of a portable ejection and injection device according to Embodiment 13 of the present invention.
Fig. 23 is a sectional view of a portable ejection and injection device according to Embodiment 14 of the present invention.
Fig. 24 is a side view of a portable ejection and injection device according to Embodiment 15 of the present invention.
Fig. 25 is a sectional view of a portable ejection and injection device according to Embodiment 16 of the present invention.
Fig. 26 is a sectional view of a portable ejection and injection device according to Embodiment 17 of the present invention.
Fig. 27 is a sectional view of a portable ejection and injection device according to Embodiment 18 of the present invention.
Fig. 28 is a sectional view of another portable ejection and injection device.
Fig. 29 is a sectional view of another portable ejection and injection device.

### Preferred Embodiments of the Invention.

The present invention is below described in details in conjunction with accompanying drawings.

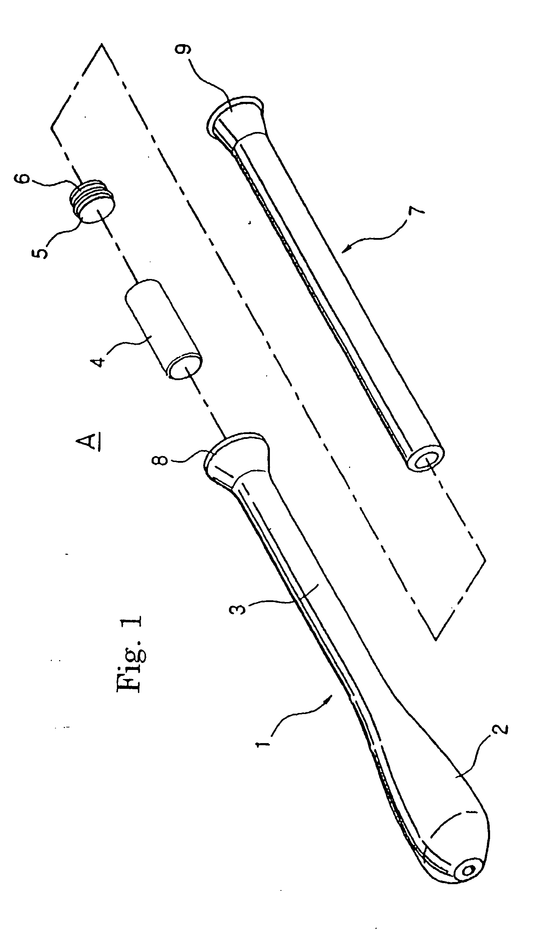

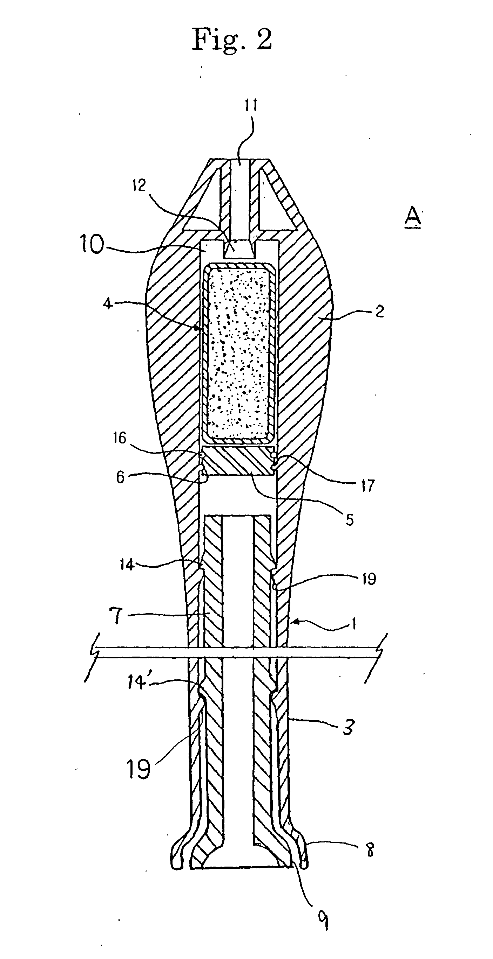

A portable ejection and injection device A according to Embodiment 1 of the present invention is illustrated in Figs. 1 to 3. Basic structure of the portable ejection and injection device A includes, as shown in Figs. 1 and 2, a cylinder 1 having an ejection nozzle 11 at the tip end and a piston member 5 therein. The piston member 5 is pushed inward and shifted toward the ejection nozzle 11 from a rear end of the cylinder 1 by means of a pushing rod 7 so as to eject a liquid medicine or a lubricant (hereinafter referred to as a medicament) through the ejection nozzle 11.

The cylinder 1 includes a insertion part 2 having a section of an circle or ellipse at a front tip end of the cylinder 1, a longitudinal part 3 linearly extending from the insertion part 2, and a funnel part 8 at a rear end of the longitudinal part 3. Within the cylinder 1 is provided a hollow portion which extends with a substantially same diameter from the ejection nozzle 11 to the rear end and in the insertion part 2 is provided a filling chamber 10 to receive a gel capsule 4 in which a medicament is filled.

The insertion part 2, as shown in Fig. 2, is formed in a streamlined shape in a manner that the shape gradually increases in diameter from the front tip toward the rear end and a diameter at the most increased position is larger than that of the longitudinal part 3. This shape allows smooth insertion into a specific body portion without pains. The insertion part 2 is provided with the ejection nozzle 11 which extends in a certain distance from the tip end of the insertion part 2 toward the rear end and ejects medicament out. The nozzle 11 is provided with a cutting portion 12 (a blade integrally formed with the ejection nozzle 11) at the rear side of the ejection nozzle 11. The filling chamber 11 is in advance filled with the gel capsule 4 containing a specific medicament through the funnel part 8 of the cylinder 1. The capacity of the filling chamber is determined by the piston member 5.

The longitudinal part include, on its inner wall, a stopping projection 19 having a section of triangle, the position of which is slightly closer toward the rear end than another later-described projection 17 (annular or non-annular) for positioning of the piston member 5. The stopping projection 19 has, in case of the projection tip being an apex angle of the triangle, an angle of a front-side slope of about 80°(a large angle) and an angle of a rear-side slope of about 20°to 30°(a small angle).

The piston member 5 is formed in a thick disc plate having plural circumferential grooves 6 on the outer circumferential face and positioned near the filling chamber 10. One of the circumferential grooves 6 of the piston member 5 receives an elastic sealing member 16 (made of elastomer), and another circumferential groove 6 located below the above groove 6 receives a projection 17 (annular or non-annular) formed on the inner wall of the cylinder 1. The piston member 5 guarantees the liquid-tightness between the inner wall of the cylinder 1 and the outer wall (face) of the piston member 5 at the time medicament being pushed out by means of the sealing member 16. Engagement between the projection 17 and the groove 6 allows the piston member 5 to be positioned in place in the cylinder 1.

The pushing rod 7, as shown in Fig. 1, is formed in a straight cylinder or rod body and is provided with an enlarged part 9 at the rear end, which is fully received in the funnel part 8 at the time of complete pushing-in.

The pushing rod 7, as shown in Fig. 2, has on the outer circumferential1 face a stopping projection 14 which engage the stopping projection 19 on the cylinder's inner wall. This stopping projection 14 has, in case of the tip being an apex angle, an angle of the front side slope of 20°to 30°(a small angle) and an angle of the rear side slope of 80°(a large angle).

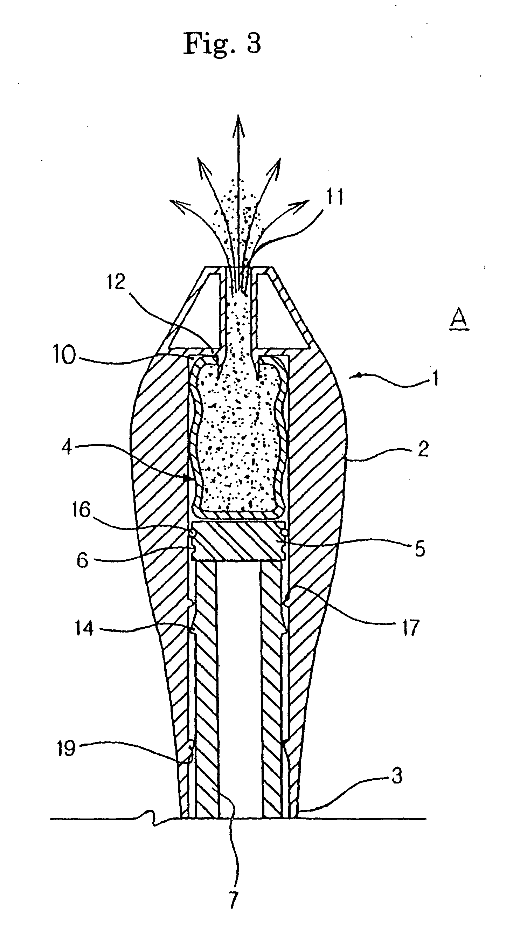

Being inserted in the longitudinal part 3 of the cylinder 1 and pushed inward, the stopping projection 14 shifts, rides on and across the stopping projection 19 and results in being supported in a certain position slightly away from the position of the piston member 5. In the portable ejection and injection device A, the filling chamber 10 is filled with the capsule 4, and when the pushing rod 7 is further pushed in toward the piston member 5, an upper portion of the capsule 4 is cut out with the cutting portion 12 and medicament is ejected out through the ejection nozzle 11, as shown in Fig. 3.

Furthermore, as shown in Fig. 2, the pushing rod 7 has another stopping projection 14' positioned nearer the rear end than the above stopping projection 14, when the stopping projection 14' shifts over and beyond the stopping projection 19, the enlarged part 9 gets in the funnel part 8 and the inward shift is completed. The stopping projection 14' has the same shape as the stopping projection 14. The stopping projection 14' can ride on and across the stopping projection 19 when the pushing rod 7 is pushed inward. However, once the stopping projection 14' shifts beyond and passes the stopping projection 19, the stopping engagement between the stopping projections 14' and 19 prevents the pushing rod 7 from coming off or allows it to hardly fall off the cylinder 1.

The medicament used in the portable ejection and injection device A includes lubricants, cleaning agents, anesthetics and the like.

With this portable ejection and injection device A, medicament can be directly injected into a female genital organ (a vagina) and an inside of a person's anus. The device is used as below.
(1) The tip portion of the cylinder 1 is inserted into a female genital organ or a person's anus. Since the tip portion of the cylinder 1 is formed in a streamlined shape tapered toward the front tip, no or little pain accompanies the insertion.
   Thereafter, the cylinder 1 is held with an index and a middle finger of a user and the pushing rod 7 is pushed inward with his or her thumb. Then, the tip portion of the pushing rod 7 reaches the piston member 5, the piston member 5 is pushed toward the front side, and the engagement between the projection 17 and the groove 6 is released,
(2) When the pushing rod 7 is further pushed forward from the above position, along with the piston member 5, the capsule 4 filled with medicament is also pushed forward and the tip portion of the capsule 4 is cut out with the cutting portion 12.
(3) When the pushing rod 7 is more further pushed forward, the medicament is ejected (or jetted) out through the ejection nozzle 1.
(4) While the above state is kept for a while, the inward shift of the pushing rod 7 is completed with the enlarged part 9 being received in the funnel part8. When all or most of the medicament contained within the capsule 4.is ejected out through the ejection nozzle 11, the stopping projection 14' passes over the stopping projection 19. As a result, the engagement between the stopping projections 14' and 19 is obtained and this engagement prevents the pushing rod 7 from falling out or allows to hardly come out cylinder 1. Consequently, the portable ejection and injection device cannot be reused. And infection of diseases is prevented and sanitary use of the device is possible.
(5) A female genital organ or a person's anus and the inside thereof can be cleaned with a jet of medicament.

With the portable ejection and injection device A constituted as stated above, a female genital organ or a person's anus and the inside thereof can be cleaning with a jet of medicament thereto, and, if an occasion demands, the medicament can be directly injected therein.

In the portable ejection and injection device A, as shown in Fig 2, a gel capsule 4 is filled in the filling chamber 10, so that leakage of medicament can be eliminated. Furthermore, in this device A, since the pushing rod 7 and the piston member 5 are separated and the piston member 5 is supported by the projection 17 and the groove 6, even the pushing rod 7 is shifted, the medicament is prevented from leaking. This advantageous working and effect can be also obtained in Embodiments 2 and 3 to be described below.

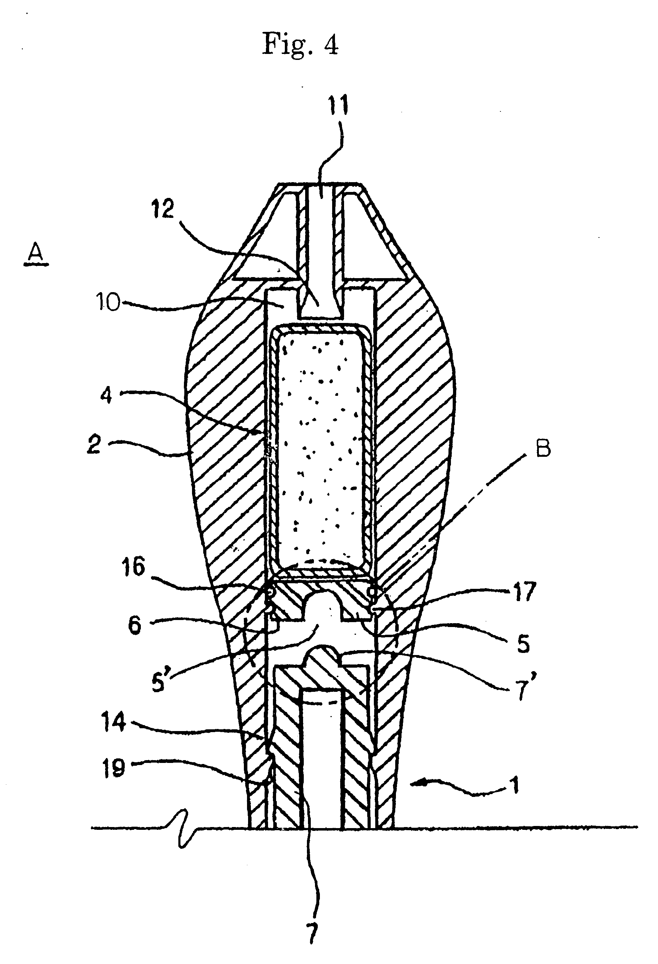

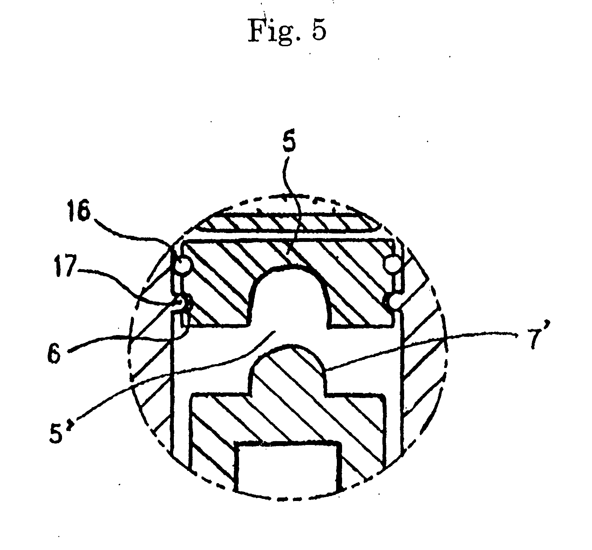

A portable ejection and injection device A according to Embodiment 2 has the same basic structure as Embodiment 1. As shown in Figs. 4 and 5, however, the structure of the piston member 5 and the pushing rod 7 is different.

In the portable ejection and injection device A of this embodiment, a bottom face (the rear side face) of the piston member 5 has a recess 5' in the center, while an top face of the pushing rod 7 has a protrusion 7' that is to be received in the recess 5'. The protrusion 7' functions like a medical syringe when medicament is filled within the filling chamber 10, allows to fill the device with a certain amount of medicament and keeps the piston member 5 at a certain position. After the filling of medicament, the piston member 5 can be removable from the pushing rod 7 by distorting the rod 7 to some extent. The recess 5' and the protrusion 7' can be threaded to be joined together.

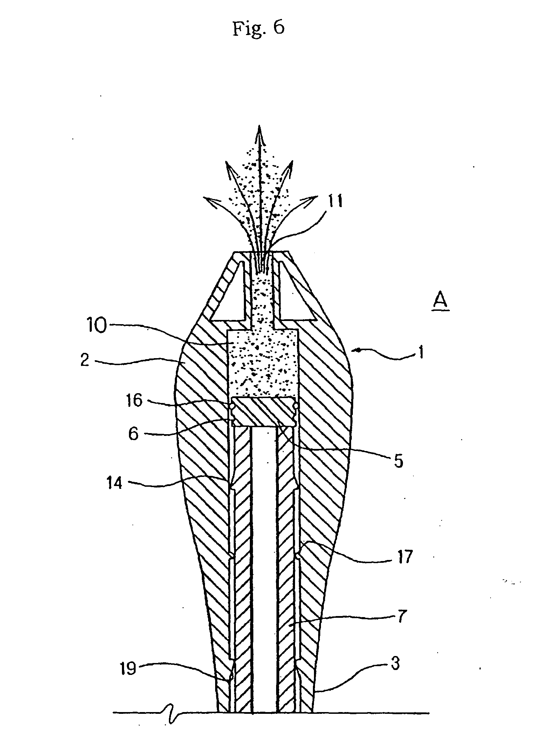

The portable ejection and injection device A according to Embodiment 3, as shown in Fig. 6, has the same basic structure as Embodiment 1. However, different from it, the ejection nozzle 11 does not have a cutting portion 12.

In this Embodiment, a prescribed capacity of the filling chamber 10 is defined and determined by the piston member 5. A certain amount of medicament is put into the cylinder with the ejection nozzle 11 being not covered, and then filled in the chamber 10 with the piston member 5 and the pushing rod 7. Alternatively, another syringe with a needle containing medicament is inserted into the cylinder 1 and medicament can be filled in the filling chamber 10 from the syringe.

As stated above, after medicament is filled in the filling chamber 10, the pushing rod 7 is pushed inward abutting against the piston member 5 due to a certain force, and thereby the medicament is jet-ejected through the ejection nozzle 11, as shown in Fig. 6.

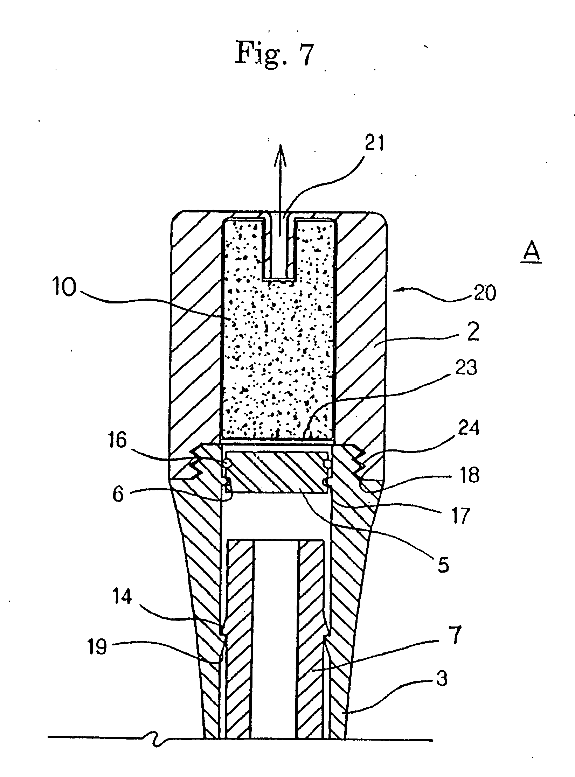

A portable ejection and injection device A according to Embodiment 4 has the same basic structure and effects as Embodiment 1. As shown in Fig. 7, different from Embodiment 1, the insertion part 2 and the longitudinal part 3 are separately formed and the two parts 2 and 3 are united together through threaded portions to produce the cylinder 1, and the insertion part 2 is formed in a cylindrical shape. In Embodiment 4, the insertion part 2 is constituted with a cap member 20. The cap member 20 has a female thread portion 18 on a rear-side inner wall, while the longitudinal part 3 has a male thread portion on a front-side outer face.

The cap member 20, as shown in Fig. 7, has a membrane member 23 at the rear and is closed therewith, an ejection nozzle 21 extending a certain length at the center of the tip portion, and the filling chamber 10 communicates with the ejection nozzle 21. After medicament is filled in the filling chamber 10, an outlet of the ejection nozzle 21 may be sealed with an adhesive tape or a cap.

In the longitudinal part 3, as shown in Fig. 7, the piston member 5 is positioned at a front end portion. Other parts and portions are same as those in Embodiment 1.

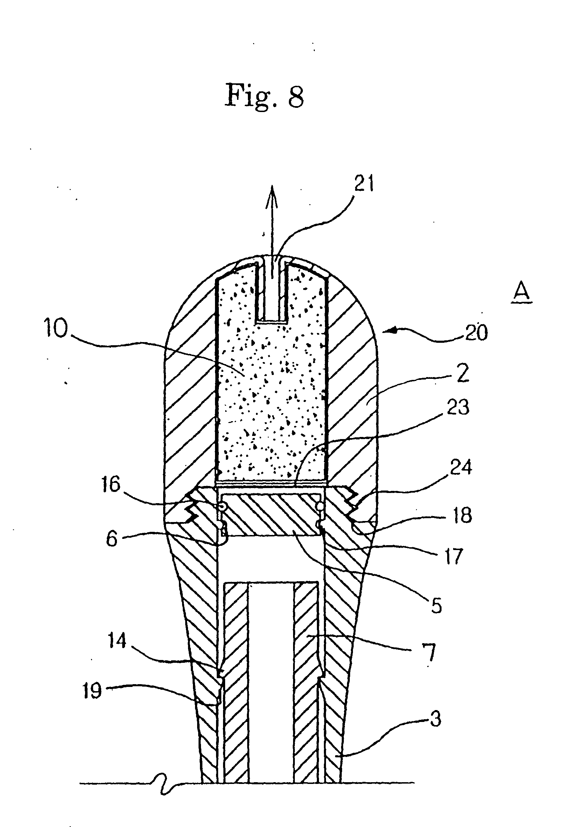

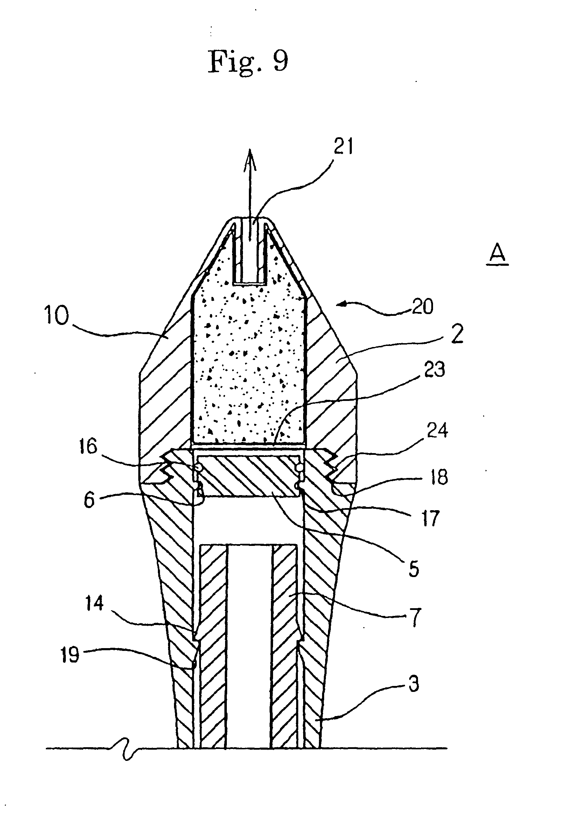

With the above mentioned structure, a variety of shapes of cap member 20 can be used according to user's preference and purposes. For examples, a cap member in a rectangular or square shape viewed from front (a cylindrical shape) as shown in Fig. 7, a cap member having a hemisphere portion on the front top side as shown in Fig. 8, a cap member with a section of triangle (a conical shape) on the front side as shown in Fig. 9, a cap member with a circular truncated cone on the front side can be employed.

In the portable ejection and injection device A, when the pushing rod 7 is pushed toward the front tip, the membrane member 23 is torn and the medicament can be ejected (jet-ejected) out through the ejection nozzle 21.

In a portable ejection and injection device according to Embodiment 4, medicament is filled within the cap member 20 the bottom open end of which is closed with the membrane member 23 and the nozzle of which is sealed with a tape. The cap member 20 can be carried around separately from the other parts such as the pushing rod 7, then leakage of medicament can be completely avoided.

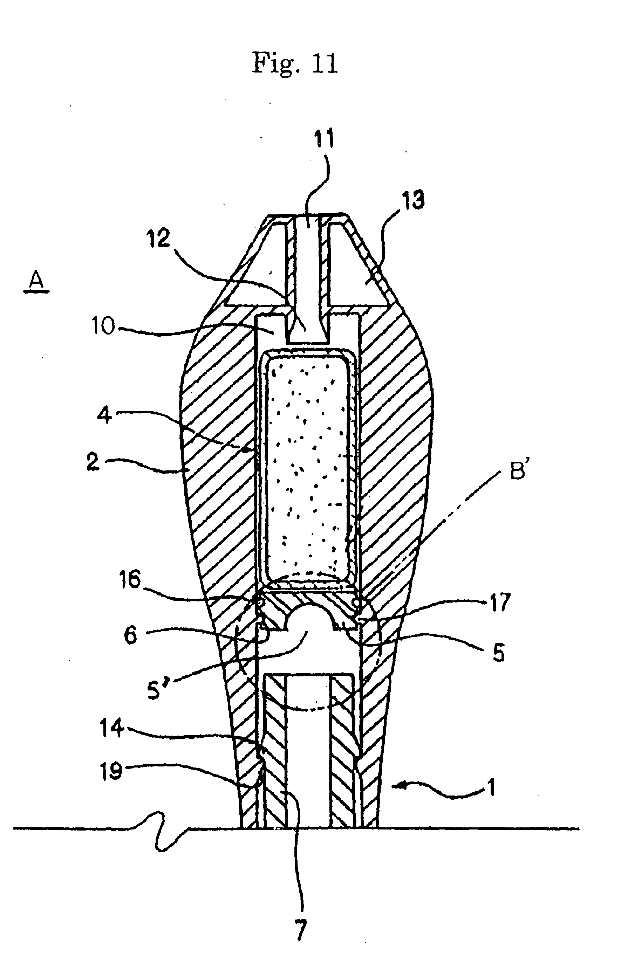

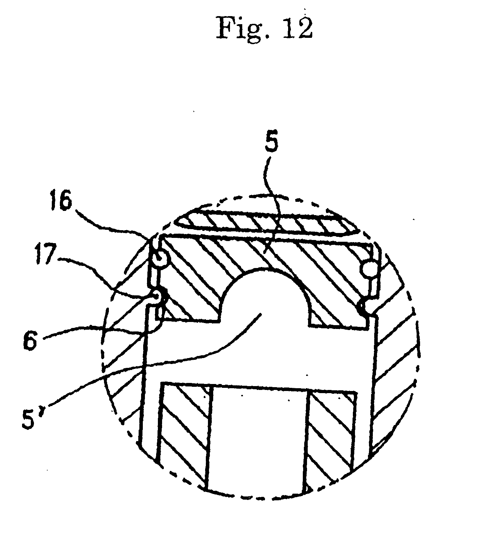

A portable ejection and injection device according to Embodiment 5 is basically constituted similarly to Embodiment 1. Unlike Embodiment 1, as shown in Figs. 11 and 12, the piston member 5 has a recess 5' in the center. In other words, the central part of the bottom of the piston member 5 is formed thinner due to the recess 5'. As a result, once the piston member 5 is pushed in, this device A cannot be reused, because the central part of the piston member 5 is thin, and even if the piston member is tried to be pushed back with a needle or the like inserted through the ejection nozzle 11, the central part of the piston member 5 has been torn and opened, and the piston member 5 cannot be pushed back.

As shown in Figs. 2, 11 and 12, since the pushing rod 7 is hollow from the top to the bottom, the needle inserted through the ejection nozzle 11 passes through the piston member 5 and then reaches the hollow portion of the pushing rod 7, and the pushing rod 7 cannot be pushed back to the position before use with the needle, either. Therefore, the portable ejection and injection device cannot be reused and may be used sanitarily, and allows infection of diseases to be prevented.

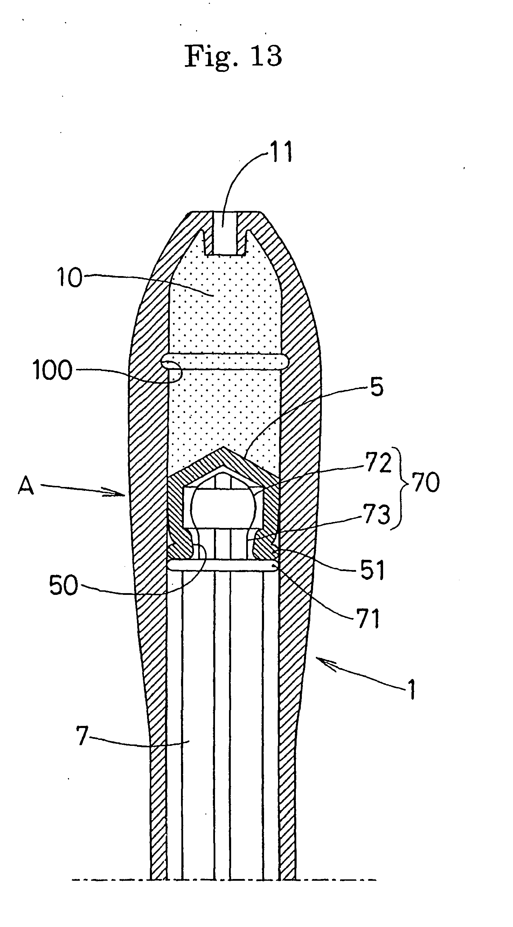

A portable ejection and injection device A according to Embodiment 6 has the same basic structure as Embodiment 1. As shown in Figs. 13 to 15, after pushing the piston member 5 to and near the ejection nozzle 11, the pushing rod 7 is pulled backward, and the piston member 5 remains at or near the ejection nozzle 11 by means of engagement with the inner wall of the cylinder 1. For this purpose, the piston member 5, the pushing rod 7 and the inner wall of the cylinder 1 are constituted as follows.

The piston member 5 is formed in a cap shape with its bottom being open as shown in Fig. 13. Stopping projections 50 and 51 are respectively provided on the inner wall and the outer circumferential face near the opening of the piston member 5.

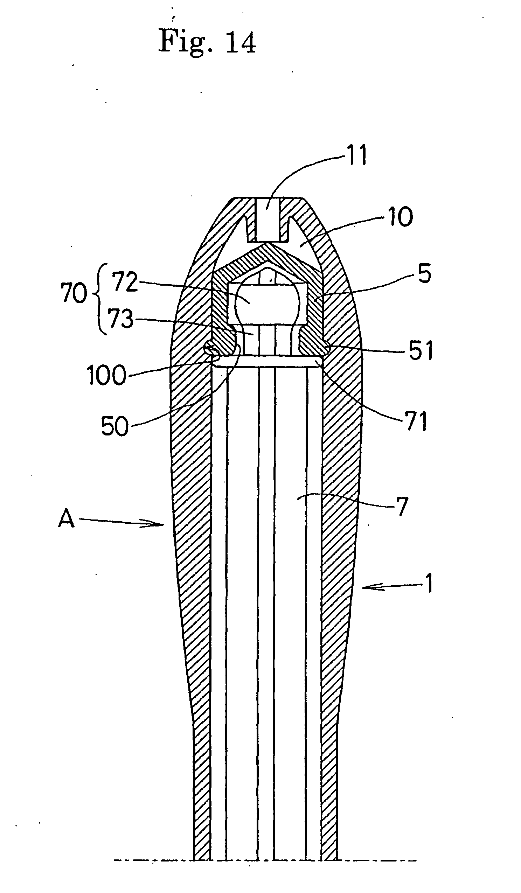

As shown in Fig. 14, the inner wall of the cylinder 1 is provided with a recess 100 into which the stopping projection 51 is received when the piston member 5 is shifted to the tip end position of the filling chamber 10 (the ejection nozzle 11 or the vicinity thereof).

As shown in Figs. 13 to 15, the pushing rod 7 has, on the tip side, a reception dish 71 to receive the opening side of the piston member 5 and a piston support 70 which extends from the reception dish 71. The piston support 70 has a head portion 72 at its tip and a neck portion the diameter of which is smaller than that of the largest portion of the head portion 72.

The cap-shaped piston member 5, as shown in Fig. 13, covers from the head portion 72 to the neck portion 73. The head portion 72 is forcibly pushed widening the stopping projection 50 and inserted within the cap-shaped piston member 5. As a result, the piston member 5 cannot come off the pushing rod 7 inadvertently. In this portable ejection and injection device A, the stopping force between the head portion 72 and the stopping projection 50 is set smaller than that between the stopping projection 51 and the constituent wall of the recess 100.

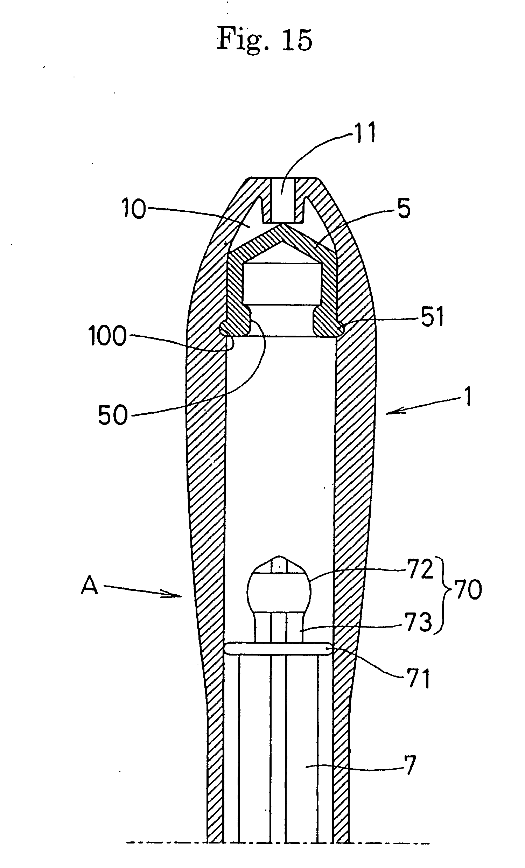

Therefore, in this portable ejection and injection device A, as shown in Fig 14, as the pushing rod 7 is pushed inward, the piston member 5 is pushed to or near the ejection nozzle 11. As shown in Fig. 15, when the pushing rod 7 is pulled back, the piston member 5 remains at or near the ejection nozzle 11. As a result this portable ejection and injection device A cannot be reused, infection of diseases can be prevented, and sanitary use can be possible.

In Embodiments 1 to 6 described above, the cylinder 1 has the ejection nozzle 11 or 21 at the tip portion, but not limitative thereto, and the ejection nozzle may be formed as a hole punched out on the tip side wall.

Below is described portable ejection and injection devices A of other Embodiments 7 to 15, which provide the following advantageous effects; medicament (in particular, of jell) does not flow or ooze out while being carried around, and with a jet of medicament to a female genital organ or a person's anus, the portion can be cleaned, and if a demand arises the medicament can be directly injected within the portion.

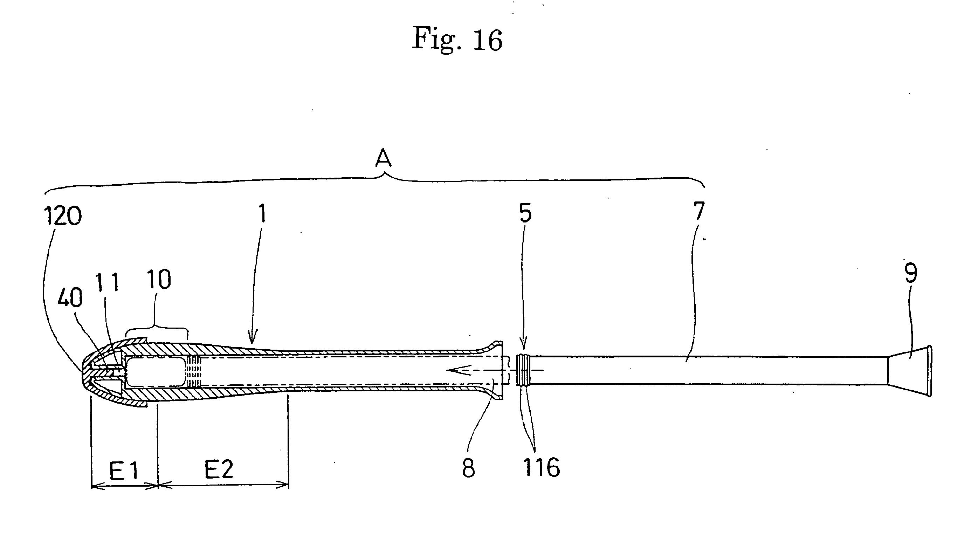

A portable ejection and injection device A of Embodiment 7, as shown in Fig. 16, has a cylinder 1 having an ejection nozzle 11 at the tip portion and a filling chamber 10 to contain medicament, a piston member 5 to seal the medicament filled in the filling chamber 10 within the cylinder 10, a pushing rod 7 integrally formed with the piston member 5, and a cap 120 to close the ejection nozzle 11.

The cylinder 1, formed cylindrically, is made of harmless ABS resin which is safe even a child licks it or puts it into the mouth. The front side part is shaped in a manner that the outer diameter gradually increases from the tip to some extent and later gradually decreases. More specifically, the front side part of the cylinder 1 has a front face with an 8mm diameter, a largest diameter of 15mm, a first section E1 extending 15mm from the front face to the position of the largest diameter, a second section E2 decreasing in diameter rearwards and extending 20mm from the rear end of E1 to the position having a 12mm diameter and the overall length of the cylinder 1 is about 90mm. The joining portion between the front face and the outer face of the cylinder body is smoothly chamfered.

The piston member 5, made of ABS resin (rubber may be employed), as shown in Fig. 16, is formed in a thick circular plate and the outer circumferential wall is provided with two O rings (sealing members) 116 at an interval. As shown by the double dotted line in Fig. 16, the piston 5 is shiftable without losing airtightness within the cylinder 1 by means of the circumferentially provided O rings.

The pushing rod 7 is made of ABS resin and formed integrally and coaxially with the piston member 5. The diameter is slightly smaller than that of the inner diameter of the cylinder 1. When the pushing rod 7 is fully pushed inward, the enlarged portion (in a truncated cone) at the rear end of the pushing rod 7 is received within the funnel portion 8 at the rear end of the cylinder 1 and the medicament within the filling chamber 10 is almost completely pushed (jetted) out through the ejection nozzle 11 by means of the piston member 5.

The cap 120 has a shape to closely cover the first section E1 of the cylinder member 1. When the cap being fitted on the cylinder 1, an axis projecting backward from the bottom face of the cap 120 is stuck in the ejection nozzle 11.

This portable ejection and injection device A is sizewise portable and plural of devices A can be carried together in a bag. Since the cap 120 has the shape to closely cover the first section E1 and the axis to be stuck in the ejection nozzle 11, while the device is being carried with the cap on, the medicament is prevented from flowing or oozing out inadvertently. The medicament is unlikely to flow out and stain the inside of the bag which the device A is in.

The portable ejection and injection device A can eject out medicament through the ejection nozzle 11 by the cylinder being held between user's index and middle fingers and the pushing rod 7 being pushed inward with user's thumb. Therefore with a jet of medicament to a female genital organ or a person's anus, such body portion can be easily cleaned, and medicament can be directly injection within the body portion, if necessary

In the portable ejection and injection device A, as stated above, since the front side portion of the cylinder 1 has the front face with the 8mm diameter, the largest diameter of 15mm, the first section E1 extending 15mm from the front face to the position of the largest diameter and the second section E2 decreasing in diameter rearwards and extending 20mm from the rear end of E1 to the position having the 12mm diameter, the overall length of the cylinder 1 is about 90mm, and the joining portion between the front face and the outer face of the cylinder body is smoothly chamfered, insertion of the device into a vagina or an anus can be done without pain, and the device is unlikely to come off inadvertently even if force from the vagina wall or the anus wall is applied.

In the above stated shape, the first and second sections E1 and E2 have a circular section, but not limitative thereto, they may have a somewhat flattened circular or elliptical section.

The cap 120 may be an adhesive tape or seal to cover an aperture of the ejection nozzle 11.

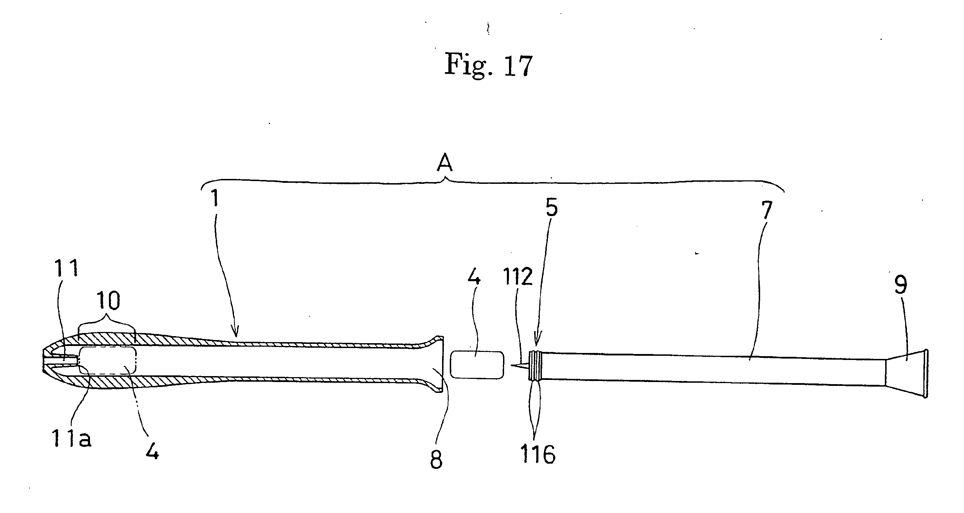

Fig. 17 is an exploded view of a portable ejection and injection device of Embodiment 8 in which the cylinder 1 is illustrated in a sectional view. A capsule 4 containing medicament is used and the capsule 4 is, in use (when ejecting medicament out), stuck and torn with a needle 112 provided on the front face of the piston member 5. With carrying the capsule 4 separately from the device without putting it in the cylinder 1, medicament can never flow out from the ejection nozzle 11. The cylinder 1 and the pushing rod 7 of Embodiment 8 have the same structure as in Embodiment 7 and the same advantageous effects can be obtained in Embodiment 8.

Instead of the needle 112, the ejection nozzle 11 may be provided with a sharp cutting portion 12a at the read end and this portion may be used for tearing the capsule 4.

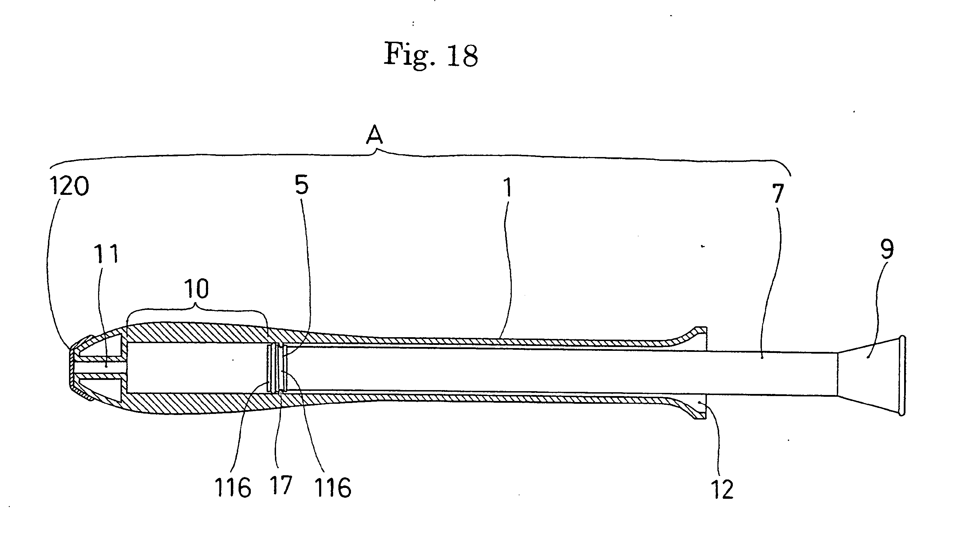

A partial sectional view of a portable ejection and injection device of Embodiment 9 is illustrated in Fig. 18. The piston member 5 has a circumferential groove 6 on the outer face and the cylinder 1 has an annular projection 17 on the inner wall which is received in the circumferential groove 6. The engagement between the groove 6 and the projection 17 causes the piston member 5 not to shift unless a force larger than a certain strength is applied on the pushing rod 7. Therefore, even if an adhesive tape or seal is used to close the ejection nozzle 11, the medicament is most unlikely to flow out from the ejection nozzle 11. The cylinder 1 and the pushing nozzle 7 of Embodiment 9 are the same as those of Embodiment 7, and the other advantageous effects can be similarly obtained in Embodiment 7.

Alternatively, an annular projection may be provided on the piston member 5, while a circumferential groove to receive the projection may be provided on the inner wall of the cylinder 1, and the same engagement therebetween as above may be obtained.

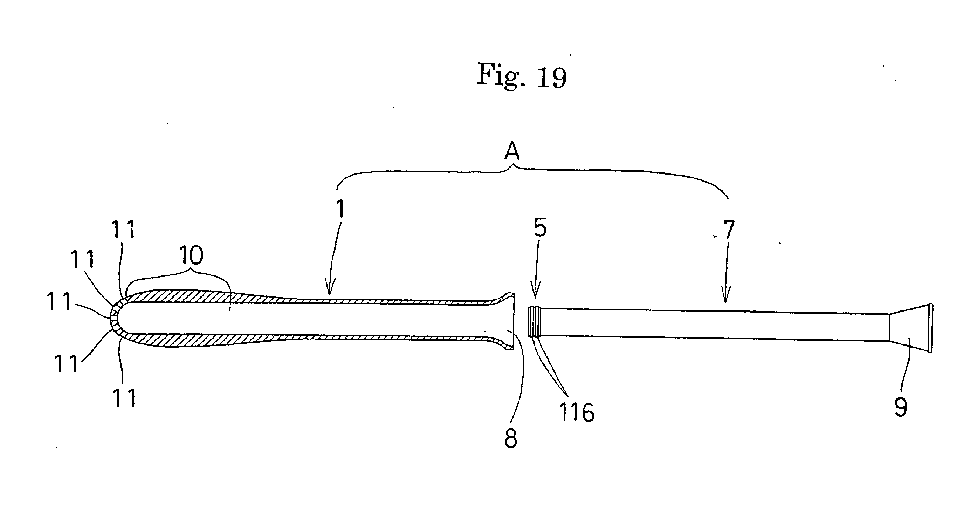

An exploded view of a portable ejection and injection device of Embodiment 10 in which a cylinder 1 is illustrated in a sectional view is shown in Fig. 19. The cylinder 1 has a plurality of ejection nozzles 11 on the front end, with which medicament can be ejected out simultaneously to a wider area. Although not shown in the drawing, the same type of cap as of Embodiment 7 is put on the front tip potion of the cylinder 1.

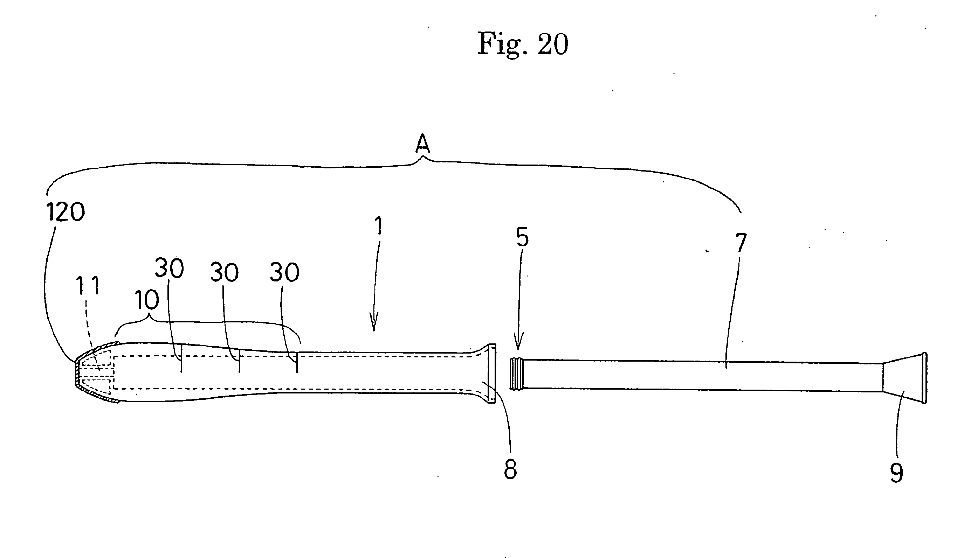

An exploded view of a portable ejection and injection device of Embodiment 11 is shown in Fig. 20. The cylinder 1 is transparent or half-transparent, has three or plural divisions 30 of a scale at the same intervals on the outer face, and contains three times of dosage of medicament. While watching the position of the front end of the piston member 5, a user can use the substantially exact amount of one division of the scale (a dosage) three times.

Alternatively, three divisions of a scale at the same intervals may be marked on the outer face of the pushing rod 7. Watching the rear end of the cylinder 1 and the scale, the medicament can be likewise used.

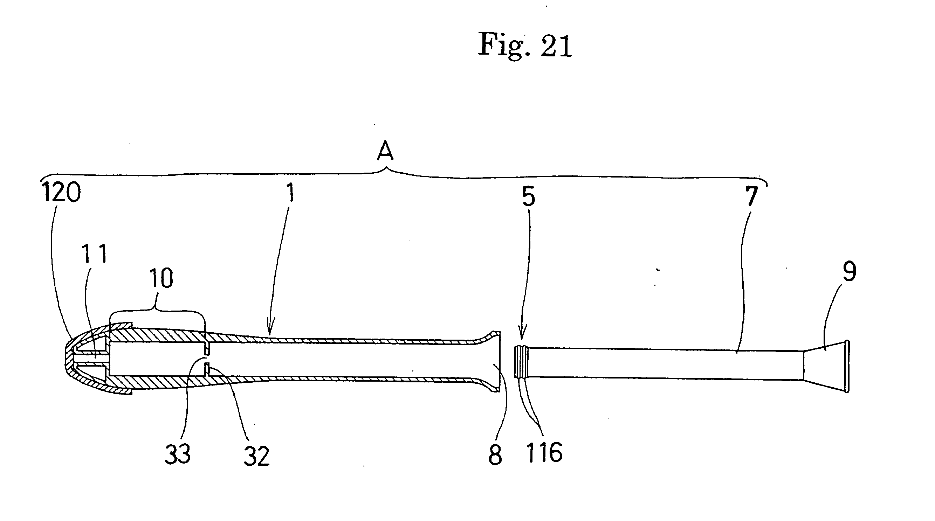

An exploded view of a portable ejection and injection device of Embodiment 12 in which the cylinder 1 is illustrated in a sectional view is shown in Fig. 21. The medicament filled in the filling chamber 10 is not directly pushed by the piston member 5, but pushed by air pressure generated by push-in of the piston member 5 by means of the pushing rod 7. For this purpose, a division wall with a central aperture 33 is provided within the cylinder 1. Shift of the piston member 5 toward the ejection nozzle 11 causes pressured air force to act on the medicament within the filling chamber 10 through the central aperture 33.

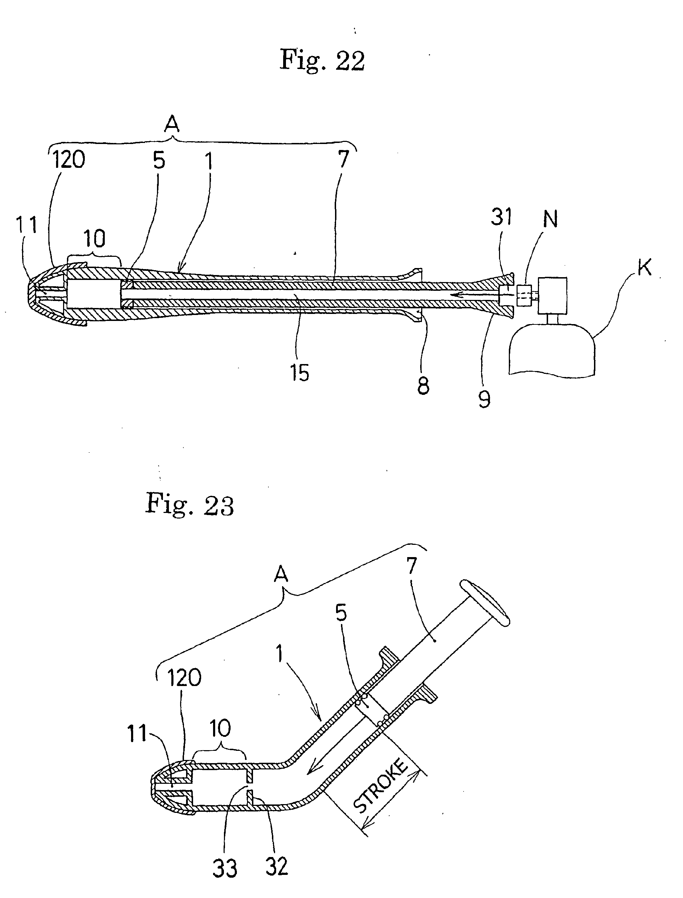

A sectional view of a portable ejection and injection device A of Embodiment 13 is shown in Fig. 22. The pushing rod 7 is formed in a cylinder to have a medicament filling passage 15. When necessary, medicament contained in an aerosol can is filled into the filling chamber 10. For this purpose, the pushing rod 7 is provided with a recess 31 on the rear end to airtightly receive an ejection nozzle N of the aerosol can, and the recess 31 is covered with a stopper (not shown) to close the recess after medicament being filled in.

The device A may has alternative structure to be filled with medicament through the ejection nozzle 11.

A sectional view of a portable ejection and injection device of Embodiment 14 is shown in Fig. 23. In addition to the foregoing advantages, in order to facilitate injection of medicament into a vagina or an inside of an anus, the cylinder 1 is bent at any position between the front end to the middle part. For this purpose, in Embodiment 14, the medicament filled in the filling chamber 10 is not pushed directly by the piston member 5 like Embodiment 6, but the medicament can be pushed out with air pressure generated by the push-in of the piston member 5 by means of the pushing rod 7. In other words, a divisional wall 32 with a central aperture 33 is provided within the cylinder 1. Shift of the piston member 5 toward the ejection nozzle 11 causes pressured air force to act on the medicament within the filling chamber 10 through the central aperture 33.

The bent angle of the cylinder 1 is not limited to the angle shown in Fig. 23, but may be adequately determined to such as 45° and 90°.

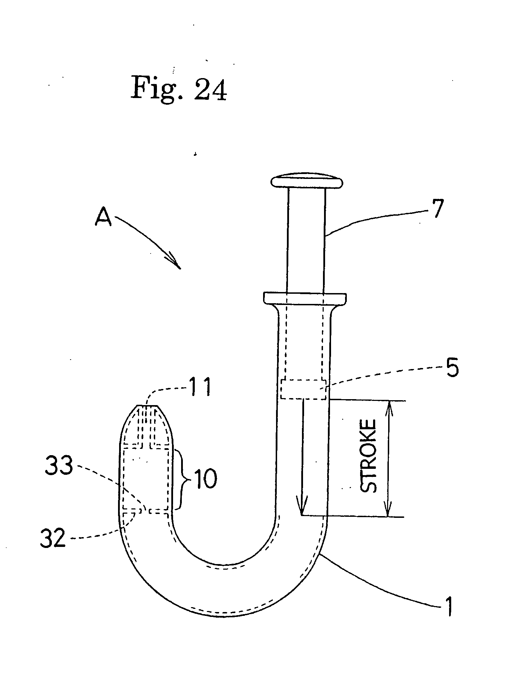

Fig. 24 Shows a side view of a portable ejection and injection device of Embodiment 15, which has the same basic structure as Embodiment 14 in Fig. 23. The difference is only that the cylinder 1 is curved in an arc shape so that the front side and the rear side of the cylinder 1 extend parallel to each other.

In above Embodiments 7 to 15, the piston member 5 and the pushing rod 7 are integral, but they may be formed separately as long as the pushing rod 7 can push the piston member 5 toward the ejection nozzle 11.

The structure of the invention which facilitates easy use in ejection and injection of medicament is described below.

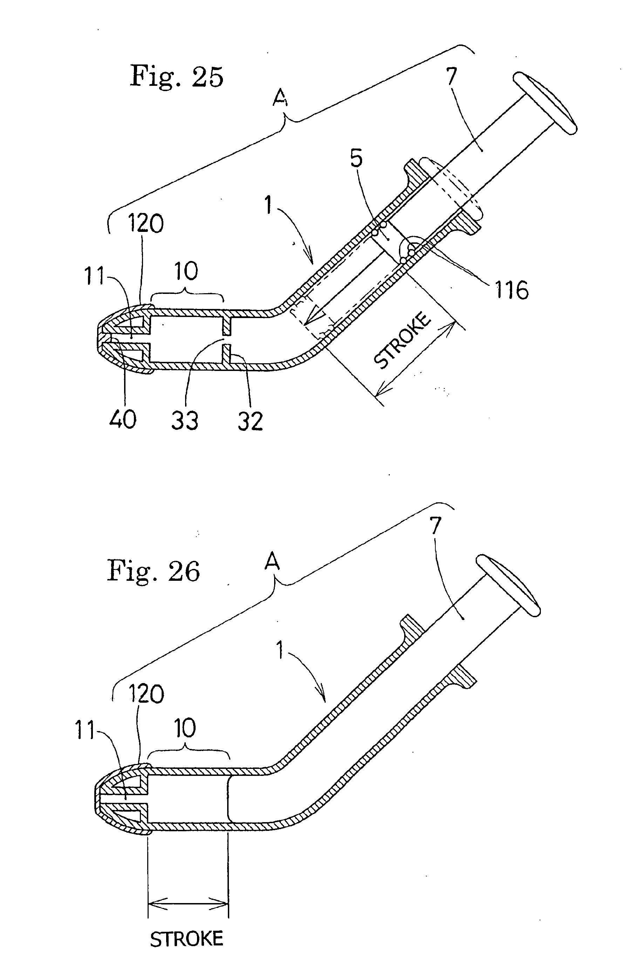

As shown in Fig. 25, a portable ejection and injection device has a cylinder 1 with an ejection nozzle 11 (a jet outlet) at the tip potion and a filling chamber 10 to contain medicament, a piston member 5 to seal the medicament filled in the filling chamber 10 within the cylinder 1, a pushing rod 7 integrally formed with the piston member 5 and a cap 120 to close the ejection nozzle 11. The cylinder is bent at the position 50mm (a range from 20 to 100mm may be possible) from the tip portion. The medicament filled within the filling chamber 10 is not pushed (spouted) out directly by the piston member 5, but spouted in use of air pressure generated by push-in of the piston member 5 by through the pushing rod 7.

The cylinder 1 is made of ABS resin which is harmless even if a child licks it or put it into the mouth, and shaped in a manner that the diameter gradually increases from the tip face of an 8mm diameter to some extent and therefrom extends rearwards with a certain diameter of about 15mm. Within the cylinder 1, as shown in Fig. 25, is provided a divisional wall 32 with a central aperture 33. Along with the shift of the piston member 5 toward the ejection nozzle 11, the medicament in the filling chamber 11 receives a pressured air force through the central aperture 33. In this cylinder 1, the joining portion between the front face and the outer circumferential face is smoothly chamfered, which avoid pain accompanying the insertion of the device into a human body.

The piston member 5 is formed in a thick disc made of ABS resin (or of rubber), and provided with two O rings (sealing members) 116 on the outer circumferential wall as shown in Fig. 25, The piston member 5 is shiftable while maintaining airtightness within the cylinder 1 by means of the outer circumferential O rings 116.

The pushing rod 7 is made of ABS resin, formed coaxially and integrally with (or separately from) the piston member 5 and has a diameter slightly smaller than that of the inner diameter of the cylinder 1 (or the diameter of a hollow portion of the cylinder 1). When being fully pushed in, the pushing rod 7 stops in front of the bent portion of the cylinder 1.

The cap 120 has a shape to closely cover the front portion of the cylinder 1 and, when being fitted on the cylinder 1, the axis 40 projecting from the bottom face of the cap 120 is stuck in the ejection nozzle 10.

Plural of the portable ejection and injection devices A can be carried in a bag in view of the size. When the cap 120 being on the cylinder 1, the axis 40 projecting from the bottom face is stuck in the ejection nozzle 11, and therefore, while the device being carried, medicament is most unlikely to flow or ooze out inadvertently, in another words, it does not happen that medicament flows out and stains the inside of the bag which the device As are in.

The rear side part of the cylinder 1 of the portable ejection and injection device A is held between user's index and middle fingers, and the rear end of the cylinder 1 is pushed inward with the user's thumb, so that medicament is spouted out through the ejection nozzle 11. Since the cylinder 1 is bent and inclined by about 30°, cleaning with and injecting of medicament can be done readily.

A portable ejection and injection device A of Embodiment 17 is illustrated in a sectional view of Fig. 26, and designed to obtain the same effects as in Embodiment 16. As shown in Fig. 26, the pushing rod 7 can be elastically bent and curved along with the shape of the cylinder 1 bent by 30°, with which the medicament is directly pushed out through the ejection nozzle 11. This is only the difference from Embodiment 16.

The pushing rod 7 may be formed a rod shape with a circular section of elastic synthetic resin, rubber, elastomer and the like or a tube shape with the both ends being closed.

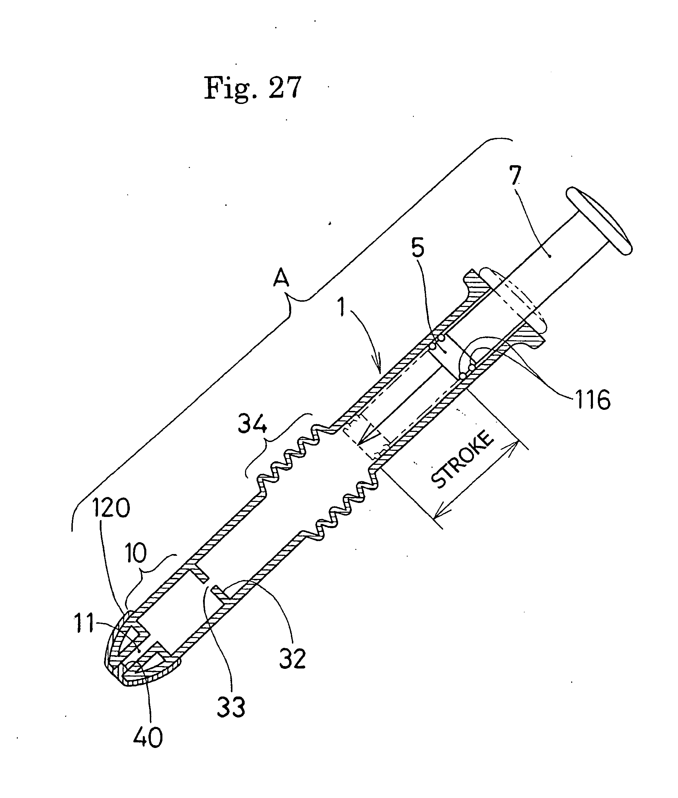

A portable ejection and injection device a of Embodiment 18 is illustrated in the sectional view of Fig. 27, and designed to obtain the same effects as in Embodiment 16. As opposed to Embodiment 16 in which the cylinder 1 is bent or curved by a certain angle, in Embodiment 18, the corresponding portion is formed with a bellow member 34 so that the bent or curved angle can be freely variable.

When the pushing rod 7 is fully pushed inward, the piston member 5 stops before reaching the bellow member 34 as shown in Fig. 27, and a jet of medicament through the ejection nozzle 11 is generated by increased air pressure within the cylinder 1. The bellow member 34 is designed to maintain the deformed shape.

The portable ejection and injection devices A stated above have a circular section, but not limitative thereto, they may have a slightly flattened circular or elliptical section.

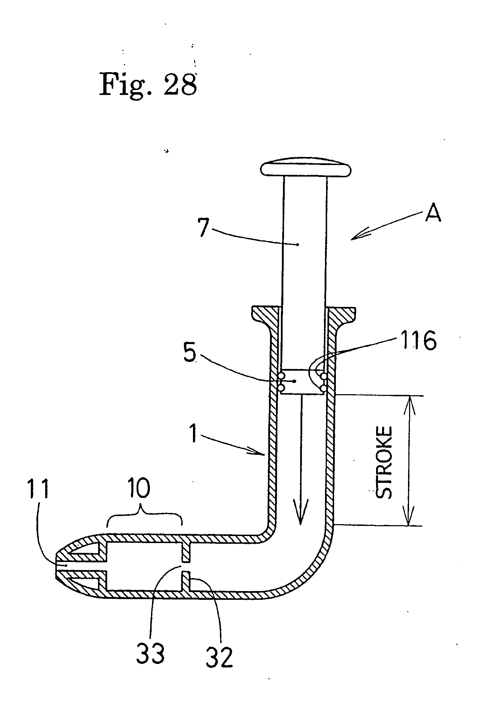

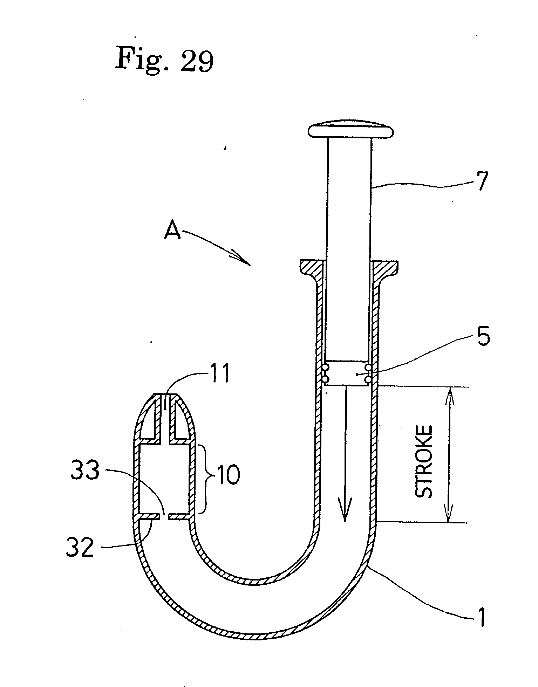

The portable ejection and injection devices A have a bent angle of 30°, but not limitative thereto, they may have a bent angle in a range from 30°to 90°(for example, 45°or 90°as shown in Fig. 28). And furthermore the devices may be curved into a U-shape, as shown in Fig. 29.

The cap 120 may be an adhesive tape or sheet to cover the opened portion of the ejection nozzle 11.

## Claims

1. An portable ejection and injection device of medicament to and into a female genital organ or a person's anus comprising a cylinder having an ejection nozzle provided at an tip portion of the cylinder and a filling chamber of medicament provided within the cylinder, a piston member to seal medicament within the filling chamber and a pushing rod inserted within the cylinder apart from the piston member to push the piston member toward the ejection nozzle when a push-in force is applied, wherein a spout of medicament is ejected through the ejection nozzle.

2. The portable ejection and injection device according to Claim 1, wherein the ejection nozzle is formed with a certain depth and provided with a cutting portion extending rearward at an rear end of the nozzle.

3. The portable ejection and injection device according to Claim 1, wherein the medicament is filled in a capsule.

4. The portable ejection and injection device according to Claim 1, an outer circumferential face of the piston member has a groove to receive a sealing member for sealing between the outer circumferential face and an inner wall of the cylinder and a groove to engage with a projection formed on the inner wall.

5. The portable ejection and injection device according to Claim 1, wherein an outer circumferential face of the pushing rod is provided with a stopping projection and the inner wall of the cylinder is provided with another stopping projection, and these stopping projections support the pushing rod within the cylinder.

6. The portable ejection and injection device according to Claim 1,wherein a bottom face of the piston is provided with a recess, and a top face of the pushing rod is provided with a protrusion to be received in the recess.

7. The portable ejection and injection device according to Claim 1, wherein the cylinder is formed with an insertion part and a longitudinal part, the insertion part is formed as a cap member having the ejection nozzle, the filling chamber and a easily breakable sealing membrane member to seal a rear end of the filling chamber, the cap member being removably joined with a thread with the longitudinal part, the longitudinal part supports the piston member therein, and when the pushing rod is pushed inward the piston member is thereby pushed in and shifts into the filling chamber breaking the membrane member.

8. The portable ejection and injection device according to Claim 7, wherein an outer circumferential face of the piston member is provided with a groove to receive a sealing member for sealing between the outer circumferential face and the inner wall of the cylinder and a groove to engage a projection provided on the inner wall of the cylinder.

9. The portable ejection and injection device according to Claim 7, wherein an outer circumferential face of the pushing rod and the inner wall of the cylinder are respectively provided with stopping projections which engage each other to support the pushing rod within the cylinder.

10. The portable ejection and injection device according to any one of Claims 1 to 9, wherein ejection of medicament through the ejection nozzle is a jet spout.

11. The portable ejection and injection device according to Claim 5, wherein, when the pushing rod is fully pushed in to a position where no further inward shift is possible, the pushing rod falls in a come-off prevention state from the cylinder by means of stopping engagement between the stopping projections respectively provided on the outer circumferential face of the pushing rod and the inner wall of the cylinder.

12. The portable ejection and injection device according to Claim 11, wherein the pushing rod falls in a come-off prevention state from the cylinder at a time when the pushing rod has been pushed in until an entire length is received within the cylinder.

13. The portable ejection and injection device according to Claim 1, wherein a part opposing to the ejection nozzle is formed thin to be easily broken.

14. The portable ejection and injection device according to Claim 13, wherein the pushing rod is longitudinally hollow from a top end to a bottom end.

15. The portable ejection and injection device according to Claim 1, wherein after the piston member has been pushed in to and near the ejection nozzle by means of the pushing rods, when the pushing rod is pulled back, the piston member remains at and near the ejection nozzle by means of engagement with the inner wall of the cylinder.

16. An portable ejection and injection device of medicament to and into a female genital organ or a person's anus comprising a cylinder with a ejection nozzle provided at an tip portion of the cylinder and a filling chamber of medicament provided within the cylinder, a piston member to seal medicament within the filling chamber, a pushing rod inserted within the cylinder to push the piston member toward the ejection nozzle, the piston member and the pushing rod being made separately or integrally, and a closing member of the ejection nozzle, wherein while the closing member is removed and a push-in force is applied to the pushing rod, the medicament is ejected through the ejection nozzle by means of shift of the piston member.

17. A portable ejection and injection device of medicament into a female genital organ or a person's anus comprising a cylinder with an ejection aperture provided at an tip portion of the cylinder and a filling chamber of medicament provided within the cylinder, and a pushing rod inserted within the cylinder in a manner that at least a part between an outer circumferential face of the pushing rod and the cylinder is watertight, the pushing rod being provided with push-in part which projects outside, wherein the cylinder is bent or curved between a top end and a rear end, medicament is ejected out through the ejection nozzle when a push-in force is applied to the pushing rod.

18. A portable ejection and injection device of medicament into a female genital organ or a person's anus comprising a cylinder with an ejection aperture provided at an tip portion of the cylinder and a filling chamber of medicament provided within the cylinder, and a pushing rod inserted within the cylinder in a manner that at least a part between an outer circumferential face of the pushing rod and the cylinder is watertight, the pushing rod being provided with push-in part which projects outside, wherein the cylinder is capable of being bent or curved between a top end and the rear end, medicament is ejected out through the ejection nozzle when a push-in force is applied to the pushing rod.

19. The portable ejection and injection device according to Claim 17, wherein the pushing rod is elastically bent or curved along with a shape of the cylinder by means of a push-in force onto the pushing rod, and the pushing rod directly ejects medicament through the ejection aperture.

20. The portable ejection and injection device according to Claim 17 or 18, wherein medicament is ejected out through the ejection aperture due to air pressure within the cylinder generated when the pushing rod is pushed inward in a range of a straight portion of the cylinder.

21. The portable ejection and injection device according to Claim 18, wherein the cylinder is provided with a bellow portion between the top and rear ends, and the bellow portion allows the cylinder to be bent or curved.
